# EUROPÄISCHE PATENTANMELDUNG

(11) **EP 1 929 948 A1**
(43) Veröffentlichungstag der Anmeldung: **11.06.2008**
(21) Anmeldenummer: 06025376.2
(22) Anmeldetag: 08.12.2006
(51) Int. Cl.: A61B 5/15

(54) **Stechgerät**

(71) Anmelder: Roche Diagnostics GmbH, 68305 Mannheim (DE); F. Hoffmann-La Roche AG, 4070 Basel (CH)
(72) Erfinder: Hartig, Herbert, 67434 Neustadt (DE); List, Hans, 64754 Hesseneck-Keilbach (DE)
(74) Vertreter: Mommer, Niels

(57) **Zusammenfassung**

Die Anmeldung betrifft ein Stechgerät zum Erzeugen einer Einstichwunde für das Gewinnen einer Körperflüssigkeitsprobe, umfassend ein gummielastisches Andruckteil (2) zum Anpressen an ein Körperteil (3), aus dem eine Körperflüssigkeitsprobe entnommen werden soll, und einen Stechelementantrieb (4) zum Antreiben eines in das Stechgerät (1) eingesetzten Stechelements (5) für eine Stechbewegung. Erfindungsgemäß ist vorgesehen, dass einen elektrischen Verformungssensor (6) zum Erfassen einer elastischen Verformung des Andruckteils (2) mittels einer elektrischen und/oder magnetischen Messung.

## Beschreibung

Die Erfindung betrifft ein Stechgerät zum Erzeugen einer Einstichwunde für das Gewinnen einer Körperflüssigkeitsprobe, umfassend ein gummielastisches Andruckteil zum Anpressen an ein Körperteil, aus dem eine Körperflüssigkeitsprobe entnommen werden soll, und einen Stechelementantrieb zum Antreiben eines in das Stechgerät eingesetzten Stechelements für eine Stechbewegung. Derartige Stechgeräte sind aus der WO 01/89383 bekannt und werden insbesondere von Diabetikern zur Bestimmung der Blutzuckerkonzentration verwendet.

Stechgeräte mit einem gummielastischen Andruckteil haben den Vorteil, sich an unterschiedlich geformte Körperteile anschmiegen zu können und dabei auf Körpergewebe rund um die Einstichwunde quer zur Einstichrichtung Druck auszuüben. Auf diese Weise kann das Austreten von Körperflüssigkeit aus einer erzeugten Einstichwunde gefördert und so die Probengewinnung für einen Benutzer erleichtert werden.

Bei dem aus der WO 01/89383 bekannten Stechgerät wird die von dem Benutzer auf das Andruckteil ausgeübte Andruckkraft durch eine Kombination einer Feder mit einem Endschalter überwacht. Auf diese Weise wird erreicht, dass ein Einstich und eine anschließende Messung nur dann durchgeführt werden, wenn der Benutzer mit einer für eine Probengewinnung ausreichenden Mindestanpresskraft auf das Andruckteil einwirkt.

Ein Nachteil des bekannten Stechgeräts mit dem beschriebenen mechanischen Andrucksensor besteht darin, dass zwar festgestellt werden kann, ob die von einem Benutzer erzeugte Anpresskraft einen für eine Probengewinnung erforderlichen Mindestanpressdruck übersteigt, jedoch eine für eine Probengewinnung ungünstig große Andruckkraft nicht oder nur mit einem großen aparativen Aufwand erkannt werden kann. Zu große Andruckkräfte können nämlich einen erzeugten Stichkanal verschließen und außerdem Körperflüssigkeit aus die Einstichwunde umgebendem Gewebe heraus drängen, so dass die Probengewinnung erschwert wird.

Ein weiterer Nachteil mechanischer Andrucksensoren, die auf einer Kombination einer Feder mit einem Schalter beruhen, besteht darin, dass Verschmutzungen der Schaltkontakte die Zuverlässigkeit beeinträchtigen und zu einem Ausfall führen können. Insbesondere bei dem aus hygienischen Gründen in regelmäßigen Abständen vorzunehmenden Austausch der gummielastischen Andruckelemente besteht eine erhöhte Gefahr der Verschmutzung von Schaltkontakten. Dies macht eine erhöhte Sorgfalt der Benutzer beim Austausch der gummielastischen Andruckteile erforderlich, die insbesondere für Benutzer, deren motorische Geschicklichkeit wegen Alter oder Krankheit eingeschränkt ist, mühsam ist und als Belastung empfunden wird.

Aufgabe der Erfindung ist es deshalb, einen Weg aufzuzeigen, wie bei einem Stechgerät der Eingangs genannten Art das Risiko eines wegen fehlerhaften Andrückens des Körperteils an das Andruckteil erfolglosen Einstichs reduziert werden kann.

Diese Aufgabe wird erfindungsgemäß durch einen elektrischen Verformungssensor zum Erfassen einer elastischen Verformung des Andruckteils mittels einer elektrischen und/oder magnetischen Messung gelöst.

Die Erfinder haben erkannt, dass bei einem Stechgerät mit einem gummielastischen Andruckteil für eine erfolgreiche Probengewinnung die Verformung des Andruckteils wesentliche bedeutsamer als der numerische Wert des auf das Andruckteil ausgeübten Anpressdrucks ist. Nur bei ausreichender Verformung des Andruckteils kann dieses sich an ein angepresstes Körperteil anschmiegen und eine Wölbung des Körperteils im Bereich der zu erzeugenden Einstichwunde umschließen, so dass dort ein erhöhter Körperflüssigkeitsdruck entsteht, der das Austreten von Körperflüssigkeit aus der Eintrittswunde unterstützt. Mit einem Verformungssensor kann deshalb zuverlässiger ermittelt werden, ob für eine Probengewinnung günstige Bedingungen vorliegen.

Ein erfindungsgemäß verwendeter elektrischer Verformungssensor hat zudem den Vorteil, wesentlich weniger verschmutzungsanfällig als mechanische Drucksensoren mit Endschaltern zu sein. Da bei einem Stechgerät mit einem elektrischen Verformungssensor keine offenen Schaltkontakte benötigt werden, besteht auch keine Gefahr, dass derartige Schaltkontakte durch Verschmutzung, insbesondere durch Blut, beeinträchtigt werden. Mit einem elektronischen Verformungssensor ist es zudem problemlos möglich, ein elektrisches Sensorsignal zu erzeugen, das neben der Information, ob die Verformung des Andruckteils eine für eine Probenentnahme erforderliche Mindestverformung übersteigt, zusätzlich eine Information darüber enthält, ob die Verformung des Andruckteils eine schädliche Obergrenze überschreitet. Ein Einstich unter Bedingungen, bei denen Köperflüssigkeit durch einen zu starken Anpressdruck aus dem für eine Probenentnahme vorgesehenen Bereich eines Körperteils weg gedrückt oder ein erzeugter Einstichkanal verschlossen wird, kann bei einem erfindungsgemäßen Stechgerät somit vermieden werden.

Weitere Einzelheiten und Vorteile der Erfindung werden anhand eines Ausführungsbeispiels unter Bezugnahme auf die beigefügten Zeichnungen erläutert. Die dabei beschriebenen Merkmale können einzeln und in Kombination zum Gegenstand von Ansprüchen gemacht werden.

Es zeigen:
- Fig. 1: ein schematisches Ausführungsbeispiel eines erfindungsgemäßen Stechgeräts mit einem an das Andruckteil angepressten Körperteil;
- Fig. 2: das Andruckteil des in Figur 1 dargestellten Ausführungsbeispiel bei geringerem Anpressdruck; und
- Fig. 3: das Andruckteil des in Figur 1 dargestellten Ausführungsbeispiel unverformt in einer Schrägansicht.

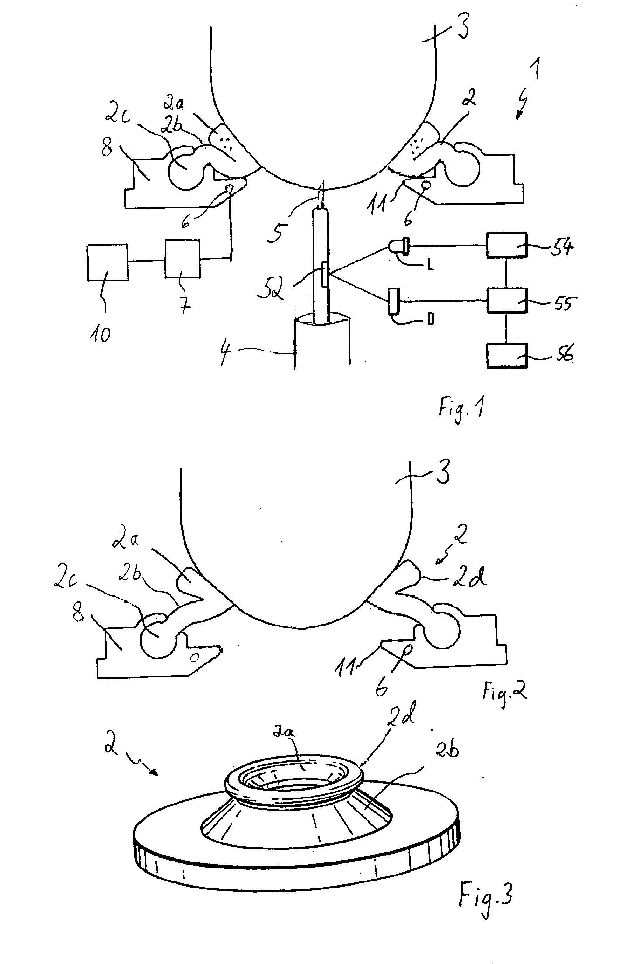

Figur 1 zeigt eine schematische Darstellung eines Ausführungsbeispiels eines Stechgeräts 1 zum Erzeugen einer Einstichwunde für das Gewinnen einer Körperflüssigkeitsprobe, umfassend ein gummielastisches Andruckteil 2 zum Anpressen an ein Körperteil 3, aus dem eine Körperflüssigkeitsprobe entnommen werden soll, und einen Stechelementantrieb 4 zum Antreiben eines Stechelements 5 für eine Stechbewegung. Das gummielastische Andruckteil ist als Andruckkonus ausgebildet, der in einem angepressten Körperteil 3 einen erhöhten Körperflüssigkeitsdruck erzeugt und so den Austritt von Körperflüssigkeit aus einer mit einem in das Stechgerät 1 eingesetzten Stechelement 5 erzeugten Einstichwunde begünstigt.

Damit sich das gummielastische Andruckteil 2 gut an ein angepresstes Körperteil 3 anschmiegen kann, hat es eine Härte von weniger als 90 Shore-A, insbesondere weniger als 50 Shore-A. Besonders günstig ist eine Shore-Härte im Bereich von 20 bis 40 Shore-A. Neben elastischen Kunststoffen, wie beispielsweise Polyurethan, sind auch Silikone und Gummi als Materialen für das Andruckteil 2 geeignet.

Das in Figur 2 in einem weniger verformten Zustand und in Figur 3 unverformt dargestellte Andruckteil 2 hat zwei konisch zulaufende Bereiche 2a, 2b, wobei der obere Bereich 2a einen sich in Anpressrichtung verengenden Andruckbereich bildet. Beim Anpressen eines Körperteils 3 an das Andruckteil 2 wölbt sich Körpergewebe in den Andruckbereich 2a hinein und wird von dem sich an das Körpergewebe anschmiegende Andruckteil 2 derart umschlossen, dass quer zur Anpressrichtung ein elastischer Druck ausgeübt wird, der eine Erhöhung des Körperflüssigkeitsdrucks in dem Bereich der zu erzeugenden Einstichwunde bewirkt.

Ein an den Andruckbereich 2a anschließender unterer Bereich 2b erweitert sich. Das Andruckteil 2 hat einen Klemmwulst 2c zur Befestigung in einer Halterung 8 des Stechgeräts 1 und einen insbesondere in Figur 3 zu erkennenden Wulst 2d am oberen Rand. Nähere Einzelheiten eines derartigen gummielastischen Andruckteils sind in der WO 01/89383 A2 offenbart, die diesbezüglich durch Bezugnahme zum Gegenstand der vorliegenden Anmeldung gemacht wird.

Eine wichtige Besonderheit des dargestellten Ausführungsbeispiels bildet ein elektrischer Verformungssensor 6 zum Erfassen einer elastischen Verformung des Andruckteils 2. Bei dem dargestellten Ausführungsbeispiel sind in Umfangsrichtung verteilt insgesamt vier derartige Verformungssensoren 6 vorgesehen, die jeweils die elastische Verformung eines Teilbereichs des Andruckteils 2 messen. Werden wie bei dem dargestellten Ausführungsbeispiel mehrere Verformungssensoren 6 eingesetzt, kann eine für eine Probengewinnung ungünstige Orientierung des Körperteils 3 zu dem angepressten Stechgerät 1 ermittelt werden, so dass die Gefahr von erfolglosen Einstichen, die nicht zu einer verwertbaren Körperflüssigkeitsprobe führen, deutlich reduziert werden kann.

Der Verformungssensor 6 erzeugt durch eine elektrische und/oder magnetische Messung ein Sensorsignal, das stetig von der Verformung des Andruckteils 2 abhängt. Auf diese Weise kann festgestellt werden, ob die durch Anpressen des Körperteils 3, aus dem eine Körperflüssigkeitsprobe entnommen werden soll, bewirkte Verformung des Andruckteils 2 in einem günstigem Bereich liegt. Dies ist deshalb bedeutsam, da eine zu geringe Verformung den Körperflüssigkeitsdruck in dem Körperteil 3 in dem Bereich der zu erzeugenden Einstichwunde in einer für eine möglichst schmerzarme Probengewinnung unzureichenden Weise erhöht. Andererseits kann eine zu starke Verformung bewirken, dass Körperflüssigkeit aus dem Bereich der Einstichwunde heraus gedrängt wird, so dass bei einer zu starken Verformung eine Probengewinnung ebenfalls erschwert sein kann.

Bei dem dargestellten Ausführungsbeispiel erfasst der Verformungssensor 6 eine mit der Verformung des Andruckteils 2 verbundene Magnetfeldänderung. Das Andruckteil 2 enthält einen magnetischen Zusatzstoff, beispielsweise Ferritpartikel oder einen anderen ferro- oder ferrimagnetischen Zusatzstoff. Magnetische Partikel können einem Kunststoff, aus dem das Andruckteil gefertigt wird, problemlos beigemischt und dauerhaft magnetisiert werden, so dass mit einer Verformung des Andruckteils 2 eine Magnetfeldänderung verbunden ist, die von dem Verformungssensor 6 erfasst werden kann. Bei dem Verformungssensor 6 kann es sich beispielsweise um einen Hall-Sensor handeln. Geeignet sind ferner auch induktive Sensoren. Ein induktiver Sensor enthält einen Schwingkreis mit einer Spule. Die Induktivität dieser Spule ändert sich, wenn sich Material mit einer hohen Permeabilitätszahl, beispielsweise feinteilige Ferrite oder in dem Andruckteil 2 eingebettete Metallpartikel durch eine Verformung des Andruckteils 2 an den Sensor 6 annähern.

Mit derartigen Verformungssensoren 6 kann eine Verformung des Andruckteils 2 kontaktfrei gemessen werden, so dass die Gefahr einer Beeinträchtigung durch Verschmutzung weitestgehend vermieden werden kann. Der Verformungssensor 6 kann geschützt in einem Bauteil des Stechgeräts 1, beispielsweise einem die Verformung des Andruckteils 2 begrenzenden Stützring 11, angeordnet oder mit einer Schutzschicht überzogen werden, so dass eine einfache Reinigung des Stechgeräts 1 ohne Gefahr einer Beschädigung des Sensors 6 möglich ist.

Eine weitere Möglichkeit zur Messung einer Verformung des Andruckteils 2 besteht darin, den Verformungssensor 6 als resistiven Sensor auszubilden. Beispielsweise kann der Verformungssensor 6 einen Dehnungsmessstreifen umfassen, der von dem Andruckteil 2 getragen wird. Da das Andruckteil 2 stark verformt wird, ist in diesem Fall darauf zu achten, dass Dehnungsmessstreifen ausreichend dehnbar ist, so dass er beim Anpressen des Andruckteils 2 an ein Körperteil nicht reißt.

Ein Dehnungsmessstreifen kann auch an der Halterung 8 des Andruckteils 2 oder dem Stützring 11 angebracht werden. Eine Verformung des Andruckteils 2 bewirkt nämlich wegen einer Übertragung des Anpressdrucks stets auch eine Verformung eines mit dem Andruckteil in Kontakt stehenden Geräteteils, die dann mit einem Dehnungsmessstreifen gemessen werden kann. Ein derartig angeordneter Dehnungsmessstreifen ist ein Kraftsensor. Ein Aspekt der Erfindung betrifft deshalb ein Stechgerät zum Erzeugen einer Einstichwunde für das Gewinnen einer Körperflüssigkeitsprobe, umfassend ein gummielastisches Andruckteil 2 zum Anpressen an ein Körperteil 3, aus dem eine Körperflüssigkeitsprobe entnommen werden soll, und einen Stechelementantrieb 4 zum Antreiben eines in das Stechgerät 1 eingesetzten Stechelements 5 für eine Stechbewegung, gekennzeichnet durch einen Dehnungsmessstreifen als Kraftsensor zum Ermitteln, ob das Andruckteil mit einer für eine Probengewinnung günstigen Andruckkraft angepresst wird.

Im einfachsten Fall kann ein Dehnungsmessstreifen als ein dünner Widerstandsdraht oder als eine dünne Folie aus einem Widerstandswerkstoff in das Andruckteil 2 eingelassen oder darauf aufgebracht werden, so dass eine Verformung des Andruckteils 2 eine Änderung des elektrischen Widerstands des Dehnungsmessstreifens bewirkt.

Tragen einzelne Bereiche des Andruckteils 2 separate Dehnungsmessstreifen, kann die Verformung einzelner Teilbereiche des Andruckteils separat erfasst werden. Werden mehrere Dehnungsmessstreifen als Dehnungsmessrosette elektrisch voneinander isoliert in mehreren Lagen mit unterschiedlicher Orientierung in das Andruckteil 2 eingearbeitet oder darauf aufgebracht, so kann vorteilhafter Weise sogar eine Information über die Richtung der Verformung gewonnen werden. Bei einer von dem Andruckteil 2 getragenen Dehnungsmessrosette kann beispielsweise eine Verformung in Andruckrichtung durch eine erste Lage der Dehnungsmessrosette erfasst werden, deren Widerstandsdrähte oder Widerstandsbänder in Andruckrichtung verlaufen. Eine Verformung quer zur Andruckrichtung kann mittels einer weiteren Lage der Dehnungsmessrosette erfasst werden, deren Widerstandsdrähte bzw. Widerstandsbänder in der entsprechenden Querrichtung verlaufen.

Von dem Verformungssensor 6 erzeugte Sensorsignale werden von einer elektronischen Auswerteeinheit 7 ausgewertet. Die Auswerteeinheit 7 ermittelt anhand der Sensorsignale, ob die Verformung des Andruckteils 2 ein für eine Probengewinnung günstiges Maß hat, beispielsweise eine vorgegebene Mindestverformung erreicht und eine vorgegebene Maximalverformung nicht übersteigt. Da der Verformungssensor 6 die elastische Verformung des Andruckteils 2 mittels einer elektrischen und/oder magnetischen Messung erfasst, kann die Verformung sogar quantifiziert werden, was gegenüber mechanischen Kraftmessern, bei denen von einer Feder ein Endschalter betätigt wird, ein großer Vorteil ist.

Werden, wie bei dem dargestellten Ausführungsbeispiel, mehrere Verformungssensoren 6 eingesetzt, die jeweils die elastische Verformung eines Teilbereichs des Andruckteils 2 messen, kann von der Auswerteeinheit 7 zusätzlich ermittelt werden, ob eine für eine Probengewinnung ungünstige Orientierung des Körperteils 3 zu dem angepressten Stechgerät 1 vorliegt. Weichen beispielsweise die von den einzelnen Verformungssensoren 6 erfassten Werte der Verformung der verschiedenen Teilbereiche des Andruckteils 2 um mehr als einen vorgegebenen Betrag, beispielsweise 30%, voneinander ab, so kann daraus geschlossen werden, dass das Körperteil 3 in einem ungünstigen Winkel gegen das Andruckteil 2 gepresst wird.

Ein von der Auswerteeinheit 7 ermitteltes Ergebnis, insbesondere ob günstige Bedingungen für eine Probengewinnung vorliegen, kann einem Benutzer mittels einer Anzeigeeinrichtung 10 angezeigt werden. Diese Anzeige kann beispielsweise als numerischer Wert, Balkendiagramm, Signallicht oder Signalton erfolgen. Im einfachsten Fall genügt beispielsweise ein grünes Signallicht zum Anzeigen günstiger Bedingungen. Eines oder mehrere weitere Signallichter können beispielsweise eine zu geringe oder zu starke Verformung signalisieren. Sind, wie bei dem gezeigten Ausführungsbeispiel, mehrer Verformungssensoren 6 vorhanden, können einem Benutzer die Informationen "Anpresskraft zu klein", "Anpresskraft zu groß", "Anpresskraft ungleichmäßig" und "Anpresskraft günstig" signalisiert werden, so dass der Benutzer die Bedingungen unter denen das Körperteil 3 an das Andruckteil 2 gedrückt wird, bei Bedarf entsprechend ändern kann.

Um die Gefahr von Fehlbedienungen zu reduzieren kann die Auswerteeinheit 7 mit dem Stechelementantrieb 4 gekoppelt werden und einen Einstich bei Vorliegen vorteilhafter Bedingungen automatisch auslösen. In einem solchem Fall kann es günstig sein, den Einstich nicht sofort bei Feststellen einer günstigen Verformung auszulösen, sondern erst dann, wenn während einer vorgegebener Zeit von beispielsweise 0,5 bis 2 s eine günstige Verformung festgestellt wird. Auf diese Weise kann verhindert werden, dass eine Probengewinnung durch Bewegungen des angepressten Körperteils 3 beeinträchtigt wird.

Da von manchen Benutzern ein automatisches Auslösen eines Einstichs aus psychologischen Gründen als unangenehm empfunden wird, kann die Auswerteeinheit 7 auch mit einer Sicherungseinrichtung (nicht dargestellt) verbunden werden, die ein Auslösen eines Einstichs verhindert, wenn nicht für eine Probengewinnung günstige Bedingungen vorliegen. Entsprechende Stechgeräte haben ein benutzerbetätigbares Auslöseelement, beispielsweise eine Taste. Erst wenn sowohl das Auslöseelement betätigt wird als auch die Sicherungseinrichtung von der Auswerteeinheit 7 freigegeben wird, erfolgt ein Einstich. Beispielsweise kann der Stechelementantrieb 4 mit einem Auslösestromkreis verbunden sein, der einen ersten von der Auswerteeinheit 7 betätigbaren Sicherungsschalter und einen zweiten von dem Auslöseelement betätigbaren Sicherungsschalter aufweist. Sind beide Schalter des Auslösestromkreises geschlossen, wird das Stechelement 5 von dem Stechelementantrieb 4 in eine Einstichbewegung versetzt.

Bei dem in Figur 1 schematisch dargestellten Stechgerät 1 handelt es sich um ein integriertes System zur Gewinnung und Analyse einer Körperflüssigkeitsprobe. Das Stechelement 5 enthält deshalb einen Kapillarkanal, der in eine Analysezone 52 mündet, die im dargestellten Ausführungsbeispiel mit Testchemikalien behandelt ist und deshalb eine von der Analytkonzentration abhängige Farbänderung erfährt. Zur Analyse wird die Analysezone 52 mit einer Lichtquelle L bestrahlt und reflektierte Strahlung von einem Detektor D detektiert. Die Lichtquelle L wird von einer Steuereinheit 54 gesteuert und das Signal des Detektors D von einer Auswerteeinheit 55 ausgewertet. Vorzugsweise regelt die Auswerteeinheit 54 auch die Steuereinheit 55. Die Auswerteeinheit 54 führte eine Auswertung des Detektorsignals durch, um die Konzentration des in der Körperflüssigkeit enthaltenen Analyten zu ermitteln. Das Analyseergebnis wird mit einer Ausgabeeinheit 56, beispielsweise einer Flüssigkristallanzeige ausgegeben.

In Figur 1 ist die Auswerteeinheit 7 zur Auswertung von Signalen des Verformungssensors 6 als eine von der Steuereinheit 54 und der Auswerteeinheit 55 separate Einheit dargestellt. Diese Einheiten können jedoch auch zusammengefasst werden, beispielsweise in einem einzigen Mikroprozessor. Entsprechend kann auch die Anzeigeeinrichtung 10 mit der Ausgabeeinheit 56 zu einer einzigen Anzeigeeinrichtung zusammengefasst werden.

### Bezugszeichenliste

- 1: Stechgerät
- 2: Andruckteil
- 2a: Andruckbereich
- 2b: unterer Bereich des Andruckteils 2
- 2c: Klemmwulst
- 2d: Randwulst
- 3: Körperteil
- 4: Stechelementantrieb
- 5: Stechelement
- 6: Verformungssensor
- 7: Auswerteeinheit
- 8: Halterung
- 10: Anzeigeeinrichtung
- 11: Stützring
- 52: Analysezone
- 54: Steuereinheit
- 55: Auswerteeinheit
- 56: Ausgabeeinheit

- L: Lichtquelle
- D: Detektor

## Patentansprüche

1. Stechgerät zum Erzeugen einer Einstichwunde für das Gewinnen einer Körperflüssigkeitsprobe, umfassend
ein gummielastisches Andruckteil (2) zum Anpressen an ein Körperteil (3), aus dem eine Körperflüssigkeitsprobe entnommen werden soll, und
einen Stechelementantrieb (4) zum Antreiben eines in das Stechgerät (1) eingesetzten Stechelements (5) für eine Stechbewegung,
**gekennzeichnet durch**
einen elektrischen Verformungssensor (6) zum Erfassen einer elastischen Verformung des Andruckteils (2) mittels einer elektrischen und/oder magnetischen Messung.

2. Stechgerät nach einem der vorhergehenden Ansprüche, **dadurch gekennzeichnet, dass** der Verformungssensor (6) ein Sensorsignal erzeugt, das stetig von der Verformung des Andruckteils (2) abhängt.

3. Stechsystem nach Anspruch 1, **dadurch gekennzeichnet, dass** der Verformungssensor (6) ein resistiver Sensor ist.

4. Stechgerät nach Anspruch 3, **dadurch gekennzeichnet, dass** der Verformungssensor (6) einen Dehnungsmessstreifen umfasst.

5. Stechgerät nach Anspruch 1 oder 2, **dadurch gekennzeichnet, dass** der Verformungssensor (6) eine mit einer Verformung des Andruckteils (2) verbundene Magnetfeldänderung erfasst.

6. Stechgerät nach Anspruch 1, **dadurch gekennzeichnet, dass** der Verformungssensor (6) ein induktiver Sensor ist.

7. Stechgerät nach einem der vorhergehenden Ansprüche, **dadurch gekennzeichnet, dass** das Andruckteil (2) einen magnetischen Zusatzstoff enthält.

8. Stechgerät nach Anspruch 7, **dadurch gekennzeichnet, dass** der magnetische Zusatzstoff ferromagnetisch oder ferrimagnetisch ist.

9. Stechgerät nach einem der vorhergehenden Ansprüche, **gekennzeichnet durch** mehrere Verformungssensoren (6), die jeweils die elastische Verformung eines Teilbereichs des Andruckteils (2) messen, um eine für eine Probengewinnung ungünstige Orientierung des Körperteils (3) zu dem angepressten Stechgerät (1) zu ermitteln.

10. Stechgerät nach einem der vorhergehenden Ansprüche, **dadurch gekennzeichnet, dass** das Andruckteil (2) derart beschaffen ist, dass sich Gewebe eines angepressten Körperteils (3) in das Andruckteil (2) hineinwölbt und von dem sich elastisch verformenden Andruckteil (2) umschlossen wird, so dass sich in dem umschlossenen Gewebe ein erhöhter Körperflüssigkeitsdruck bildet.

11. Stechgerät nach einem der vorhergehenden Ansprüche, **dadurch gekennzeichnet, dass** der der Verformungssensor (6) die Verformung des Andruckteils (2) kontaktfrei erfasst.
